# EUROPEAN PATENT APPLICATION

(11) **EP 3 705 127 A2**
(43) Date of publication of application: **09.09.2020**
(21) Application number: 18873842.1
(22) Date of filing: 23.10.2018
(51) Int. Cl.: A61K 36/537, A61K 33/00, A61K 33/32, A61K 33/30, A61K 33/34, A23L 33/105

(54) **COMPOSITION COMPRISINGSALVIA MILTIORRHIZA**

(30) Priority: 31.10.2017 KR 20170143582; 19.10.2018 KR 20180125449
(71) Applicant: Huen Co.,Ltd., Asan-Si, Chungcheongnam-do 31538 (KR)
(72) Inventor: YOON, Joo Seog, Suwon-Si Gyeonggi-do 16707 (KR); KIM, Nu Ree, Cheonan-Si Chungcheongnam-do 31176 (KR); SON, Jeong Hwan, Daejeon 35016 (KR)
(74) Representative: Gulde & Partner
(86) International application number: PCT/KR2018/012522
(87) International publication number: WO 2019/088541

(57) **Abstract**

Disclosed is a composition for preventing, ameliorating or treating visceral fat obesity containing a *Salvia miltiorrhiza* radix extract having excellent activity of reducing visceral fat.

Disclosed is a composition for preventing, ameliorating or treating visceral fat obesity containing an extract of *Salvia miltiorrhiza* radix that has efficacy specific for visceral fat when administered to obese model animal mice that have obesity caused by decreased leptin secretion and diet-induced obesity (DIO) mice in which obesity is induced using a high-fat diet.

Disclosed is a composition for preventing, ameliorating or treating visceral fat obesity containing an S. *miltiorrhiza* extract, wherein the S. *miltiorrhiza* extract inhibits the production of triglyceride (TG) in liver tissue and the blood, indicating that the S. *miltiorrhiza* extract has inhibitory activity on visceral-fat-type obesity, and the S. *miltiorrhiza* extract decreases the expression of ALT, LDH, BUN, FFA or Hb_{A1C} in the blood and reduces visceral fat, as can be seen from MRI and MRS.

## Description

### BACKGROUND OF THE INVENTION

### Field of the Invention

The present invention relates to a composition for preventing, ameliorating or treating visceral fat obesity containing an extract of *Salvia miltiorrhiza* radix having an excellent activity of reducing visceral fat, and more particularly to a composition for preventing, ameliorating or treating visceral fat obesity containing an extract of *Salvia miltiorrhiza* radix that exhibits efficacy specific for visceral fat when administered to obese model animal mice that have obesity caused by deteriorated leptin secretion and diet-induced obesity (DIO) mice in which obesity is induced using a high-fat diet.

### Description of the Related Art

The recent improvement in living standards caused by economic development has brought about accumulation of excessive calorie (energy) in the body due to frequent intake of food and switching to a meat-based diet, and the change in diet as well as reduced consumption of calorie energy due to lack of exercise have caused a rapid increase in the obesity population.

Obesity is a considerably dangerous disease that may cause not only constipation, indigestion and gastrointestinal disorders due to the abdominal pressure by adipose tissue, but also may significantly increase the onset of diseases, representatively type 2 diabetes, hypertension, coronary artery disease, stroke and various cancers.

Fat, which is the main cause of such obesity, acts as a storage that stores excess energy in the body and supplies the same when needed. Fat may be broadly divided into subcutaneous fat and visceral fat. Subcutaneous fat maintains a constant body temperature and acts as an insulator, providing resistance to cold. Generally, as the proportion of subcutaneous fat increases, resistance to cold increases. In addition, subcutaneous fat absorbs and retains moisture, thereby keeping the skin moist and soft. Subcutaneous fat functions as a carrier of vitamins such as vitamins A, D, E and K and is used as a precursor of sex hormones.

However, unlike subcutaneous fat, which performs positive functions, visceral fat triggers insulin resistance, which is not only a fundamental cause of metabolic diseases such as type 2 diabetes, hypertension and fatty liver, but also a secondary cause of vascular diseases such as heart disease and stroke, dementia, hearing loss, neurodegenerative diseases such as arthritis, and aging.

Metabolic Syndrome, which is directly associated with visceral fat, is a syndrome that is accompanied by risk factors such as hypertriglyceridemia, hypertension, abnormal glucose metabolism, abnormal coagulation and obesity.

According to the ATP III of the National Cholesterol Education Program (NCEP), published in 2001, when a patient has one of five risk factors such as ① abdominal obesity with a waist circumference of 40 inches (102 cm) for men and 35 inches (88 cm) for women, ② 150 mg/dL or more of triglycerides, ③ HDL cholesterol of 40 mg/dL or less for men and of 50 mg/dL or less for women, ④ blood pressure of 130/85 mmHg or more, and ⑤ fasting glucose of 110 mg/dL or more, the patient is determined to have a metabolic disease.

As factors known to be related to causes and treatment of metabolic syndrome, exercise, dietary habits, weight, blood sugar, triglycerides, cholesterol, insulin resistance, adiponectin, leptin, AMPK activity, sex hormones such as estrogen, genetic factors, concentration of malonyl-CoA *in vivo* and the like are directly or indirectly involved therein.

The best ways to alleviate the symptoms of metabolic syndrome are known to be exercise, dietary control and weight loss. The common mechanism that this method exerts effects for metabolic syndrome is to promote energy metabolism, consume as much excess energy in the body as possible, and avoid accumulation of excess energy.

In order to effectively remove excess energy, it is indispensable to increase metabolic activity. For this purpose, it is necessary to activate factors involved in the inhibition of fat synthesis, suppression of glucose generation (glucogenesis), promotion of glucose consumption, promotion of fat acidization and the generation promotion and activation of mitochondria, which are key to energy metabolism. The activation factors related to metabolism promotion include AMP-activated protein kinase (AMPK), peroxisomal proliferator-activated receptor gamma coactivator 1-α (PGC-1α), glucose transporters (GLUT 1 and 4), carnitine palmitoyl transferase 1 (CPT1), uncoupling proteins (UCP-1, 2, 3), acetyl CoA carboxylase (ACC I, II), and the like. These play a pivotal role in energy metabolism.

Meanwhile, recently, NQO1 enzyme is emerging as a new therapeutic target for metabolic diseases. NQO1 (NAD(P)H quinone oxidoreductase) is a cytosol enzyme that has been reported to be distributed in most cells and tissue, and it is known that the expression rate thereof increases in those afflicted with diseases such as obesity, fatty liver, metabolic syndrome or cancer. NQO1 is a phase II enzyme that protects cells from chemicals *in vitro,* neutralizes toxins such as quinone and benzene, and uses NA(P)H as a cofactor. When NQO1 uses as a co-substrate NADH to neutralize quinine or benzene or oxygen peroxide species (ROS), it oxidizes the NADH to NAD⁺. This pharmacological mechanism induces cytosolic energy uncoupling to change the *in vivo* redox status, which is expressed in the intracellular ratio of NAD+/NADH, and thereby activate glucose metabolism, mitochondrial biosynthesis and fat metabolism. The increase in the intracellular ratio of NAD+/NADH induces genetic changes during long-term caloric restriction and exercise, such as activation of AMPK, which is an energy consumption mechanism for changes in the intercellular energy environment, expression of sirtuin family genes, which are anti-aging and anti-stress mechanisms, and expression of PGC1a, which activates the energy metabolism of mitochondria.

No drugs for treating metabolic syndrome have been developed to date, and there is no solution for selectively treating visceral fat, which is a direct cause of metabolic syndrome. Only drugs for treating diabetes, hyperlipidemia and hypertension have been used to treat metabolic syndrome, but have limitations.

As currently available drugs, metformin and thiazolidinediones (TZD) used to treat diabetes, and glucosidase inhibitors, dual PPARγ/α agonist and DDP (dipeptidyl peptidase) IV inhibitors used to treat diabetes, are receiving a great deal of attention. In addition, as therapeutic agent targets for blood pressure and hyperlipidemia, ApoA-I isoforms, related peptides and cholesterol ester transport protein (CETP) inhibitors are also attracting attention.

However, these drugs effectively lower cholesterol and blood lipid levels, but have a disadvantage of causing side effects. As representative side-effects, nausea, headaches, abdominal pain, diarrhea and constipation may occur, bile acid blockers have low intestinal absorption rate of vitamins A, D and K, and fibric acid derivatives are drugs inadequate for kidney disease, liver disease or gallbladder disease. Therefore, there is increasing demand for the development of medicines and functional foods containing natural products that are capable of replacing these drugs.

In addition, weight loss, which is a common method of solving obesity, refers to a decrease in the body's mass (weight), such as body water, body fat, muscle or other tissue mass. Unintentional weight loss means weight loss in the absence of arbitrary control of weight such as dietary control or exercise.

However, this unintentional weight loss may be attributed to weight loss due to hyperthyroidism, depression and physical and mental illness.

Recently, body fat is considered to be the main cause of obesity, which is a state of excessive accumulation of stored fat. In order to solve this, a healthy diet is prevalent, while maintaining muscle strength and body water through body fat reduction rather than weight loss.

A body fat percentage, meaning the ratio of body fat to body weight, is normally 10 to 20% for men and 18 to 28% for women. The ratios of visceral fat and subcutaneous fat greatly vary from person to person, depending on the degree of obesity and the amount of exercise. However, adult obesity is often due to excess visceral fat and thus an increased abdominal fat rate.

Obesity caused by visceral fat is known as the cause of adult diseases such as hypertension, cardiovascular disease and diabetes. This is because subcutaneous fat is far from the center of the human body and thus oxygen is delivered by the microvascular vessels, while visceral fat is connected to relatively large blood vessels and thus has high fat mobility.

However, most currently available obesity treatments, diet supplements and body fat reducers do not distinguish between fat and protein and degrade proteins more easily than fat, and many supplements reduce subcutaneous fat more than visceral fat.

Therefore, the present applicant has devised a composition for preventing, alleviating or treating visceral-fat-type obesity which has no side effects using an extract of *Salvia miltiorrhiza Bunge* and is effective for visceral fat.

*Salvia miltiorrhiza Bunge* is a herbal material in the traditional Chinese medicine that belongs to the genus *Salvia* of the Lamiaceae family, and in the traditional Chinese medicine, *Salvia miltiorrhiza Bunge* tastes bitter, is slightly cold, and enters the heart and liver where it has the effects of removing extravasated blood to facilitate blood circulation, cooling the blood to treat symptoms caused by heat, and cooling the blood to aid in sedation. In particular, *Salvia miltiorrhiza Bunge* is a medicinal material that removes extravasated blood from the human body and thereby improves blood circulation. It is linked to alleviation of limb joint pain, menstrual irregularities, dysmenorrhea, postpartum abdominal pain, hyperlipidemia, heart disease and brain disease, and thus is known to have pharmaceutical activity thereon, acts on cardiovascular and various vascular diseases, and has inhibitory activity against damage to the liver, lungs and kidneys, activity against osteoporosis, activity on the central nervous system, and anti-inflammatory, antioxidant and anti-cancer activities.

Meanwhile, a prior art document, Korean Patent No. 10-0818586 discloses a drug for treating obesity and metabolic syndrome containing a tanshinone derivative for promoting metabolic activity as an active ingredient. Claim 1 discloses a composition for preventing or treating metabolic syndrome containing, as an active ingredient, a mixture of at least one selected from the group consisting of (a) cryptotanshinone, tanshinone I and 15,16-dihydrotanshinone I, and (b) tanshinone IIA, wherein a weight ratio of the remaining component (a), based on the tanshinone IIA (b), is 10:1 to 1:10, the composition contains the mixture in a pharmacologically effective amount, and the composition is capable of preventing or treating one or more metabolic syndrome disease selected from the group consisting of obesity, diabetes, arteriosclerosis, hypertension, hyperlipidemia, liver disease, stroke, myocardial infarction, ischemic disease and cardiovascular disease by increasing 5'AMP-activated protein kinase (AMPK) activity.

However, the present inventors have performed research and discovered that an extract of *Salvia miltiorrhiza* radix has activity of increasing the ratio of NAD+/NADH in the cytoplasm and nucleus by acting as a substrate of NQO1 to oxidize NADH in the cytoplasm to NAD, and has metabolic activity of enabling the reduced substrate to transfer electrons to the electron transport system of mitochondria, thereby promoting the production of APT and causing recycling reaction. Furthermore, the present inventors have found that the extract of *Salvia miltiorrhiza* radix has efficacy of reducing visceral fat by administering the *Salvia miltiorrhiza* radix extract *in vivo* in order to demonstrate the efficacy of preventing, alleviating or treating visceral-fat-type obesity. The present invention has been completed based on these findings.

### SUMMARY OF THE INVENTION

Therefore, the present invention has been made in view of the above problems, and it is one object of the present invention to provide a composition for preventing, ameliorating or treating visceral fat obesity containing an extract of *Salvia miltiorrhiza* radix having efficacy specific for visceral fat.

It is another object of the present invention to provide a composition for preventing, ameliorating or treating visceral fat obesity containing an extract of *Salvia miltiorrhiza* radix that has efficacy specific for visceral fat when administered to obese model animal mice that have obesity caused by decreased leptin secretion and diet-induced obesity (DIO) mice in which obesity is induced using a high-fat diet.

In accordance with the present invention, the above and other objects can be accomplished by the provision of a composition for preventing, ameliorating or treating visceral fat obesity containing a *Salvia miltiorrhiza* radix extract.

The *Salvia miltiorrhiza* radix extract may be extracted with any one solvent selected from the group consisting of water, alcohols having 1 to 6 carbon atoms and a mixed solvent thereof.

The *Salvia miltiorrhiza* radix extract may inhibit production of triglycerides (TGs) in liver tissue and blood, and reduces expression of alanine aminotransferase (ALT), glutamic pyruvate transaminase (GPT), lactate dehydrogenase (LDH), blood urea nitrogen (BUN), free fatty acid (FFA) or glycosylated Hb (Hb_{A1C}) when administered to high fat diet-induced obesity (DIO) mice.

The *Salvia miltiorrhiza* radix extract may cause visceral fat reduction and fat loss in liver tissue as observed through magnetic resonance imaging (MRI) and magnetic resonance spectroscopy (MRS) of fat and oil red O staining of liver tissue.

The composition may contain a mixture of the *Salvia miltiorrhiza* radix extract and at least one mineral selected from chromium, manganese, magnesium, zinc, copper or calcium.

The *Salvia miltiorrhiza* radix extract may prevent, alleviate or treat a disease mediated by visceral fat selected from the group consisting of hypertriglyceridemia, fatty liver, hypertension, diabetes, hyperlipidemia, cardiocerebrovascular disease, abnormal glucose metabolism, abnormal blood coagulation or obesity.

In accordance with another aspect of the present invention, provided is a heath functional food composition for preventing or ameliorating visceral fat obesity containing a *Salvia miltiorrhiza* radix extract as an active ingredient.

The heath functional food composition may contain the *Salvia miltiorrhiza* radix extract in an amount of 0.1 to 50% by weight.

### BRIEF DESCRIPTION OF THE DRAWINGS

The above and other objects, features and other advantages of the present invention will be more clearly understood from the following detailed description taken in conjunction with the accompanying drawings, in which:
FIG. 1 shows changes in triglycerides in blood and liver tissue when administering a *Salvia miltiorrhiza* radix extract to C57BL/6JL Lep ob/Lep ob, genetically obese model animals;
FIG. 2 shows changes in blood pyruvate transaminase (GPT), lactate dehydrogenase (LDH) and blood urea nitrogen (BUN) when the *Salvia miltiorrhiza* radix extract is administered to C57BL/6JL Lep ob/Lep ob, genetically obese model animals;
FIG. 3 shows changes of *in vivo* visceral fat and liver tissue fat when the *Salvia miltiorrhiza* radix extract is administered to C57BL/6JL Lep ob/Lep ob, genetically obese model animals;
FIG. 4 shows decreases in glutamic pyruvate transaminase (GPT), lactate dehydrogenase (LDH) and blood urea nitrogen (BUN) in the blood when administering the *Salvia miltiorrhiza* radix extract to diet-induced obesity (DIO) mice in which obesity is induced using a high-fat diet;
FIG. 5 shows changes of *in vivo* visceral fat and liver tissue fat when the *Salvia miltiorrhiza* radix extract is administered to diet-induced obesity (DIO) mice in which obesity is induced using a high-fat diet; and
FIG. 6 shows changes of TG in liver tissue and blood and free fatty acid and HbA1C in the blood when the *Salvia miltiorrhiza* radix extract is administered to diet-induced obesity (DIO) mice in which obesity is induced using a high-fat diet.

### DETAILED DESCRIPTION OF THE INVENTION

The terms or words used in the present specification and claims should be construed, not as having ordinary or dictionary meanings, but as having meanings and concepts that are consistent with the technical ideas of the present invention, based on the principle that an inventor can appropriately define the concept of a term in order to describe the invention in the best way.

Therefore, it should be understood that the configurations shown in the embodiments and drawings described in the present specification are suggested only as the most preferred embodiments of the present invention and do not represent all of the technical ideas of the present invention, and various equivalents and alterations thereof that can replace them at the time of the present application are possible.

### Example 1. Preparation of extract of Salvia miltiorrhiza radix

Extraction from *Salvia miltiorrhiza* radix includes a) extracting the *Salvia miltiorrhiza* radix in water or an organic solvent to obtain a crude extract, b) filtering the crude extract and concentrating the same (under reduced pressure) and c) concentrating and drying the extract and preparing the same in various formulations.

### (1) Extracting salvia miltiorrhiza radix

In this step, a *Salvia miltiorrhiza* radix extract is obtained with hot water or 20 to 100 wt% ethanol. Depending on the design conditions, other solvents may be used, and the extraction temperature, extraction time, amount of solvent, and method of dealing with residual ingredients may be set differently according to the type of extraction solvent. The extraction solvent may be selected from among a variety of solvents, and useful extraction solvents include water, ethanol, methanol, fatty oil, glycerin, hempseed oil, ethyl acetate, acetone, butanol, isopropanol and methylene chloride.

### (2) Concentrating Salvia miltiorrhiza radix extract

In this step, moisture is removed from the extract by heating the extract at 40 to 80°C through microbar drying. Alternately, the extract is concentrated and dried through low-temperature vacuum drying. Low-temperature vacuum drying is a drying method conducted by maintaining the pressure inside a dryer in a vacuum and adjusting the temperature to about 5 to about 15°C. The method does not denature the extract components and maintains taste and aroma. Depending on the design conditions, methods such as spray drying, cold-air drying, hot-air drying, freeze drying, far-infrared drying, shade drying and reduced-pressure drying may be used.

### (3) Concentrating and drying extract to prepare variety of formulations

The extract is concentrated and dried through a spray-drying method and is then prepared into a variety of formulations. For example, the extract may be prepared as a powder. Low-temperature vacuum drying is a drying method conducted by maintaining the pressure inside a dryer in a vacuum and adjusting the temperature to about 5 to about 15°C. The method does not denature the extract components and maintains taste and aroma. Depending on the design conditions, methods such as spray drying, cold-air drying, hot-air drying, freeze drying, far-infrared drying, shade drying and reduced-pressure drying may be used.

Preferably, S. *miltiorrhiza* was eluted with 50 L of ethanol for 24 hours and concentrated under reduced pressure. 1,500 ml of water was added to the resulting concentrate and the same amount of n-hexane, dichloromethane (CH₂Cl₂) and ethyl acetate (EtOAc) were added thereto and extracted twice to prepare an S. *miltiorrhiza* extract as a red gel.

The present invention will be described in detail with reference to examples, but these examples should not be construed as limiting the scope of the present invention.

### Experimental Example 1. Analysis of S. miltiorrhiza extract

Nuclear Magnetic Resonance (NMR) analysis was performed to determine the structures of cryptotanshinone, tanshinone I, tanshinone IIA, and 15, 16-dihydrotansinone I, which are the active ingredients of the S. *miltiorrhiza* extract extracted in Example 1. Other tanshinones such as salvianolic acid, tanshinone IIB, hydrotanshinone and tanshinoic acid are contained in addition to the four tanshinones described above in the extract.

### Cryptotanshinone

1H-NMR (CDCl3) : δ 7.42 (2H, ABq, J=8.0 Hz), 4.83 (1H, t, J=9.2 Hz), 4.31 (1H, dd, J=9.2 and 6.0 Hz), 3.55 (1H, m), 3.17 (2H, br t), 1.65 (4H, m), 1.40 (3H, d, J=6.8 Hz), 1.28 (6H, s) 13C-NMR (CDCl3) : δ 9.58 (C-1), 19.00 (C-2), 37.73 (C-3), 34.76 (C-4), 143.57 (C-5), 132.48 (C-6), 122.43 (C-7), 128.30 (C-8), 126.19 (C-9), 152.28 (C-10), 184.16 (C-11), 175.59 (C-12), 118.21 (C-13), 170.66 (C-14), 81.38 (C-15), 34.54 (C-16), 18.74 (C-17), 31.85 (C-18), 31.80 (C-19)

### Tanshinone 11A

1H-NMR(CDCl3, 300.40MHz) δ 7.63(1H, d, J=8.2Hz), 7.54(1H, d, J=8.2Hz), 7.22(1H, s), 3.18(2H, t, J=6.6Hz), 2.26(3H, s), 1.78(2H, m), 1.65(2H, m), 1.31(6H, s). 13C-NMR(CDCl3, 75.45MHz) δ 184.29, 176.43, 162.38, 150.80, 145.14, 141.96, 134.13, 128.12, 127.16, 121.81, 120.91 120.57, 38.52, 35.33, 32.51, 30.56, 19.79, 9.46.

### 15, 16-Dihydrotanshinone I

1H-NMR(CDCl3, 300.40MHz) δ 9.24(1H, d, J=10.6Hz), 8.24(1H, d, J=10.3Hz), 7.69(1H, d, J=10.3Hz), 7.54(1H, dd, J=10.6, 8.4Hz), 7.41(1H, d, J=8.4Hz), 4.95(1H, t, J=11.3Hz), 4.41(1H, dd, J=11.3Hz, 7.5Hz), 3.62(1H, m), 2.66(3H, s), 1.38(3H, d, J=8.1Hz). 13C-NMR(CDCl3, 75.45MHz) δ 184.26, 175.67, 170.56, 142.00, 134.95, 134.72, 132.06, 131.90, 130.38, 128.81, 128.18, 126.01, 124.99, 120.28, 118.32, 114.06, 81.62, 34.68, 19.85, 18.81.

### Tanshinone I

1H-NMR(CDCl3, 300.40MHz) δ 9.19(1H, d, J=10.6Hz), 8.23(1H, d, J=10.3Hz), 7.73(1H, d, J=10.3Hz), 7.50(1H, dd, J=10.6, 8.5Hz), 7.30(1H, d, J=8.5Hz), 7.26(1H, q, J=1.3Hz), 2.64(3H, s), 2.25(3H, d, J=1.3Hz). 13C-NMR(CDCl3, 75.45MHz) δ183.38, 175.55, 161.13, 142.00, 135.18, 133.58, 132.90, 132.69, 130.63, 129.57, 128.31, 124.73, 123.03, 121.72, 120.43, 118.69, 19.84, 8.79.

### Experimental Example 2. Visceral fat reduction effect of S. miltiorrhiza extract in genetically obese model animals, C57BL/6JL Lep ob/Lep ob

As genetically obese model animals, C57BL/6JL Lep ob/Lep ob 8-week-old mice were left in an animal-rearing room under environmental conditions of an average room temperature of 20°C ± 2°C, a relative humidity of 60% ± 10%, and light/dark intervals of 12 hours. Each animal was bred in a well-ventilated plastic cage. Feed (purina) and straw litter were provided and periodically replaced and drinking water was freely supplied. Before beginning animal experiments, the animals were acclimated for an acclimatization period of a week to adapt to the environment of the rearing room, and were then subjected to the experiments at 8 weeks old.

After the acclimatization process, the *S*. *miltiorrhiza* extract was administered to the mice at 100 to 1000 mg/kg. The mice were freely fed with a feed (low-fat diet) which was mixed with 0.47% (corresponding to 400 mg/kg) and 0.7% (corresponding to 600 mg/kg) for 2 weeks.

In the blood biochemical test, triglycerides (TG) and enzymes present in hepatocytes, mainly alanine aminotransferase (ALT), glutamic pyruvate transaminase (GPT), blood urea nitrogen (BUN) and lactate dehydrogenase (LDH), which are released into the blood and then exhibit increased levels in the blood when hepatocytes are damaged, have standard values of 100 to 225 lU/L. They are enzymes that act when the sugar in cells is converted into energy and are abnormally oversecreted during cell damage. Changes in the distribution of visceral fat were measured through MRI, changes in visceral fat were measured through MRS, and changes of fat content of liver tissue were observed through Oil red O staining.

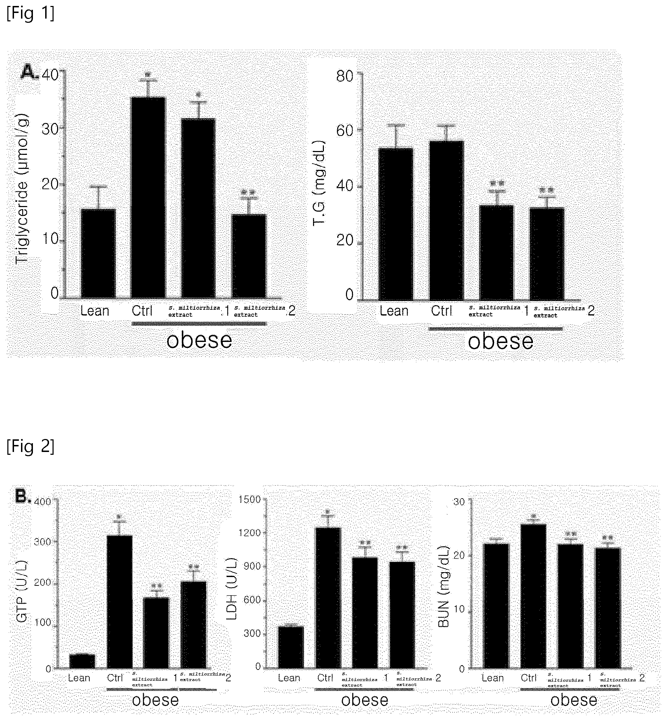

FIG. 1 shows changes in triglycerides in blood and liver tissue when administering the S. *miltiorrhiza* extract to C57BL/6JL Lep ob/Lep ob, genetically obese model animals. As can be seen from FIG. 1, compared to the control group, administration of the S. *miltiorrhiza* extract decreases triglycerides in the blood and liver tissue in a concentration-dependent manner.

FIG. 2 shows changes in blood pyruvate transaminase (GPT), lactate dehydrogenase (LDH) and blood urea nitrogen (BUN) when the S. *miltiorrhiza* extract is administered to C57BL/6JL Lep ob/Lep ob. The administration of the S. *miltiorrhiza* extract decreases the levels of GPT, BUN and LDH, which are produced due to cell damage, in the blood in a concentration-dependent manner.

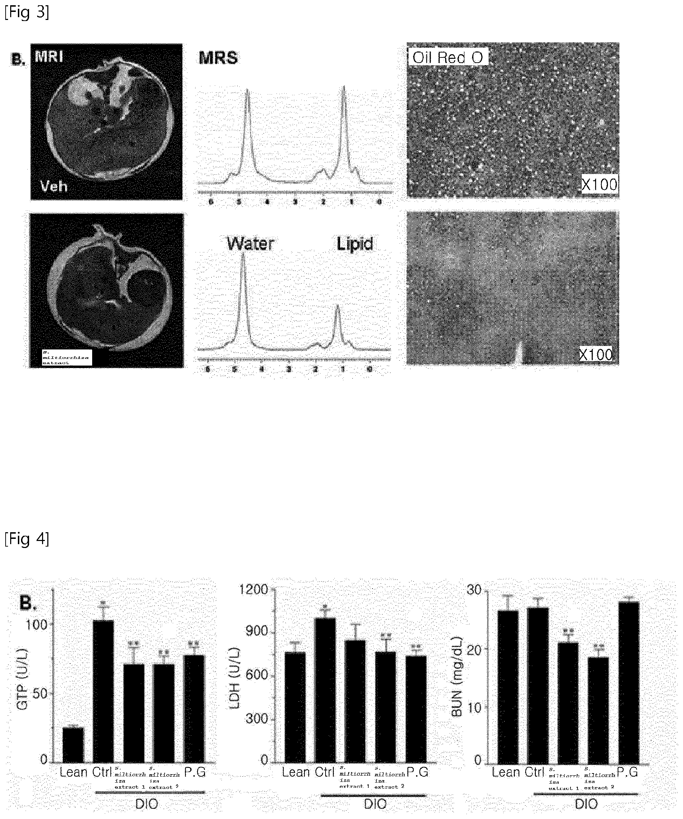

FIG. 3 shows changes in *in vivo* visceral fat and liver tissue fat when the S. *miltiorrhiza* extract is administered to C57BL/6JL Lep ob/Lep ob. As shown in FIG. 3, the results of MRI and MRS measurements showed that the experimental group administered with the S. *miltiorrhiza* extract significantly decreased the distribution of visceral fat and the result of measurement of liver tissue by Oil red O staining showed that fat content was decreased.

### Experimental Example 3. Visceral fat reduction effect of S. miltiorrhiza extract in high-fat-diet-induced obese model animals, DIO (diet-induced obesity)

As high-fat-diet-induced obese model animals (DIO; diet-induced obesity), C57BL/6 4-week-old mice were left in an animal-rearing room under environmental conditions of an average room temperature of 20°C ± 2°C, a relative humidity of 60% ± 10%, and light/dark intervals of 12 hours. Each animal was bred in a well-ventilated plastic cage. Feed (purina) and straw litter were provided and periodically replaced, and drinking water was freely supplied. Before beginning animal experiments, the animals were acclimated for a week as an acclimatization period for adapting to the environment of the rearing room. After the acclimatization process, obesity was induced using a high-fat diet for 12 weeks, and 100 to 600 mg/kg of the S. *miltiorrhiza* extract was administered orally thereto once daily for 4 weeks.

As a result, fat was excessively accumulated in the body, which maintained about 30 to 45 g, which is 1.4 times the typical weight of a mouse at about 3 months after birth. Therefore, oral administration was performed for 4 weeks to determine the effects of the *S*. *miltiorrhiza* extract on visceral fat. The results of analysis of the weights of the experimental group and the control group are shown in FIGS. 4, 5 and 6.

FIG. 4 shows decreases in GPT (glutamic pyruvate transaminase), LDH (lactate dehydrogenase), and BUN (blood urea nitrogen) in the blood when administering S. *miltiorrhiza* extract to high-fat-diet-induced obese model animals (DIO; diet-induced obesity). The administration of the *S*. *miltiorrhiza* extract decreases the levels of GPT, BUN and LDH, which are produced due to cell damage, in the blood in a concentration-dependent manner.

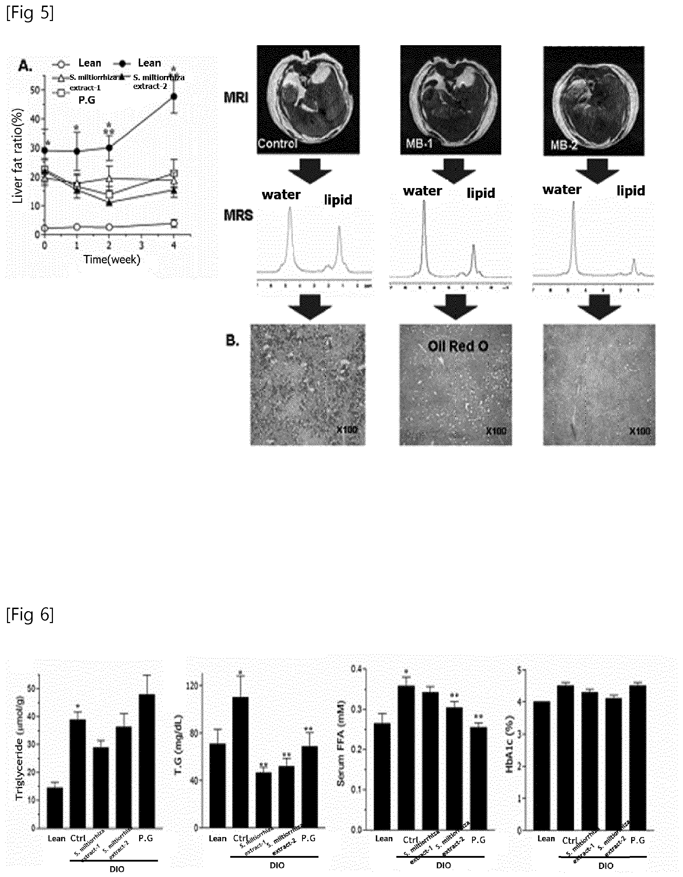

FIG. 5 shows changes of visceral fat *in vivo* when the S. *miltiorrhiza* extract is administered to high-fat-diet-induced obese model animals (DIO; diet-induced obesity). The results of MRI and MRS measurements showed that the experimental group administered with the S. *miltiorrhiza* extract significantly decreased the distribution of visceral fat compared to the control group and the result of measurement of liver tissue by Oil red O staining showed that the amount of fat in the liver was decreased.

FIG. 6 shows changes of TG in liver tissue and blood, and free fatty acid and HbA1C in the blood when the *S*. *miltiorrhiza* extract is administered to high-fat diet-induced obese animal models, DIO (diet-induced obesity). The experimental group administered with the *S*. *miltiorrhiza* extract decreased the content of TG in liver tissue and the blood and decreased the levels of free fatty acids and HbA1C in a concentration-dependent manner.

### Experimental Example 4. Specific clinical example of S. miltiorrhiza extract.

In order to determine the efficacy of the *S*. *miltiorrhiza* extract on visceral fat obesity, experiments were conducted using the obtained *S*. *miltiorrhiza* extract on patients diagnosed with visceral fat obesity based on visceral obesity diagnostic criteria. A total of 15 patients were analyzed for visceral fat obesity. The *S*. *miltiorrhiza* extract was continuously administered to the patients twice a day in the morning and evening for 3 months, and total fat, subcutaneous fat and visceral fat were evaluated.

**Table 1**

| Abdominal fat decrease rate | | | | | | |
|---|---|---|---|---|---|---|
| Item | S. miltiorrhiza extract | | | Control group (placebo) | | |
| | N | Median | Visceral fat decrease (%) | N | Median | Visceral fat decrease (%) |
| Total fat | 7 | -9.41 | -14.33 ± 13.37 | 8 | -0.69 | -0.05 ± 10.76 |
| Visceral fat | 7 | -22.65 | -18.84 ± 15.96 | 8 | -0.69 | 0.14 ± 15.05 |
| Subcutaneous fat | 7 | -8.01 | -12.06 ± 15.10 | 8 | -2.85 | -0.35 ± 10.87 |

**Table 2**

| Fat decrease rate | | | | | | |
|---|---|---|---|---|---|---|
| Item | S. miltiorrhiza extract | | | Control group (placebo) | | |
| | N | Median | Visceral fat decrease (%) | N | Median | Visceral fat decrease (%) |
| Total fat | 7 | -6.84 | -9.92 ± 7.22 | 8 | 0.69 | 0.65 ± 4.04 |
| Total Tissue | 7 | -5.41 | -6.74 ± 4.57 | 8 | -0.98 | -0.53 ± 2.39 |
| Total Mass | 7 | -3.45 | -3.45 ± 3.91 | 8 | 1.04 | 1.09 ± 2.13 |

The result of clinical trials conducted for three months to determine the efficacy of the *S*. *miltiorrhiza* extract on visceral-fat-type obesity showed that visceral-fat-type obesity was decreased by as much as 18%, as shown in Table 1.

### Example 2 Formulations containing S. miltiorrhiza extract

Experimental Examples 1, 2, and 3 showed efficacy in preventing, alleviating and reducing visceral-fat-type obesity in genetically obese model animals, ob/ob mice and high-fat diet-induced DIO (diet-induced obesity) mice. Therefore, it is possible to develop a variety of compositions for preventing, alleviating and treating visceral fat-type obesity containing the *S*. *miltiorrhiza* extract as an active ingredient.

In addition, it is possible to improve the efficacy of reducing visceral-fat-type obesity by mixing the *S*. *miltiorrhiza* extract with one or more bivalent minerals selected from chromium, manganese, magnesium, zinc, copper or calcium. The present invention will be described in detail with reference to examples, but these examples should not be construed as limiting the scope of the present invention.

### (1) Pharmaceutical formulations

The composition for preventing, alleviating or treating visceral-fat-type obesity according to the present invention may contain an *S*. *miltiorrhiza* extract and may be prepared along with a pharmaceutically acceptable carrier, if necessary.

Suitable dosages of the pharmaceutical composition according to the present invention may vary depending on factors such as the formulation method, administration mode, and age, weight, gender, morbidity, food condition, administration time, administration route, excretion rate and responsiveness of the patient.

The pharmaceutical composition for preventing, improving or treating visceral-fat-type obesity of the present invention can be administered orally or parenterally during clinical administration and can be used in the form of a general pharmaceutical formulation. That is, the composition of the present invention may be administered in various oral and parenteral formulations upon actual clinical administration, and is prepared using diluents or excipients such as fillers, extenders, binders, wetting agents, disintegrating agents and surfactants that are commonly used.

Solid formulations for oral administration include tablets, pills, powders, granules, capsules and the like, which are prepared by mixing the *S*. *miltiorrhiza* extract with at least one excipient such as starch, calcium carbonate, sucrose, lactose and gelatin. In addition to simple excipients, lubricants such as magnesium stearate and talc are also used.

Liquid formulations for oral administration are suspensions, solutions, emulsions and syrups, and may include various excipients such as wetting agents, sweeteners, fragrances and preservatives, in addition to commonly used simple diluents such as water and liquid paraffin.

Formulations for parenteral administration include sterile aqueous solutions, non-aqueous solvents, suspensions, emulsions, lyophilized preparations and suppositories. As the non-aqueous solvent and the suspension solvent, propylene glycol, polyethylene glycol, vegetable oil such as olive oil, injectable esters such as ethyl oleate and the like can be used. As a suppository base, Witepsol, macrogol, tween 61, cacao butter, laurin butter, glycerol, gelatin and the like can be used.

A dosage unit may contain, for example, 1, 2, 3 or 4 times, or 1/2, 1/3 or 1/4 times an individual dosage.

The individual dosage preferably contains an amount in which the effective drug is administered at one time, which usually corresponds to all, 1/2, 1/3 or 1/4 times the daily dose. The effective dose of the *S*. *miltiorrhiza* extract is concentration-dependent, but is preferably 0.1 mg to 1,000 mg/kg, more preferably 0.4 to 500 mg/kg, and can be administered 1 to 6 times a day. Therefore, the *S*. *miltiorrhiza* extract can be administered in the range of 0.1 to 6,000 mg/day per kg of adult body weight.

### (2) Health functional food composition

The present invention provides a health functional food composition for preventing and alleviating visceral-fat-type obesity containing an *S*. *miltiorrhiza* extract as an active ingredient.

As used herein, the term "health functional food" refers to food that improves the function of general food by adding an *S*. *miltiorrhiza* extract to the general food, and can be prepared by adding the *S*. *miltiorrhiza* extract to the general food, or through encapsulation, powderizing, suspension or the like. Ingestion of this product has a certain effect on health and has an advantage of causing no side effect that may occur when taking medicine for a long time, since it is produced from food as a raw material, unlike general medicine.

### (3) Food composition

When the *S*. *miltiorrhiza* extract of the present invention is used as a food additive, the *S*. *miltiorrhiza* extract can be added as it is, can be used in combination with other food or food ingredients, or can be appropriately used in accordance with other conventional methods. The content of the active ingredient can be suitably determined according to the use purpose thereof (prevention, health or therapeutic treatment).

In general, in the preparation of food or beverages mixed with the S. *miltiorrhiza* extract, the extract may be added in an amount of 0.0001 to 50% by weight, preferably 0.1 to 25% by weight, based on the total weight of the raw material. However, in the case of prolonged administration for health, hygiene, or health control purposes, the amount may be adjusted below the range defined above. In addition, when the health food of the present invention is used as the pharmaceutical composition, it preferably contains the *S*. *miltiorrhiza* extract within the measured toxicity range.

There is no particular limitation as to the kind of food. Examples of foods to which the *S*. *miltiorrhiza* extract may be added include meat, sausage, bread, chocolate, candies, snacks, confectionery, pizza, ramen, other noodles, gum, milk powder, Sunsik (Korean ready-to eat food), raw foods, lactobacillus fermented milk, and dairy products including ice cream, various soups, beverages, teas, drinks, alcoholic beverages, vitamin complexes and the like. Specifically, the health food containing the *S*. *miltiorrhiza* extract may include health foods and favorite products such as extract, tea, jelly and juice, which are produced from the *S*. *miltiorrhiza* extract as a main ingredient, and folk remedies for the treatment of edema, nephritis and urethritis.

### (4) Cosmetic composition

When the *S*. *miltiorrhiza* extract of the present invention is used as a cosmetic raw material, the *S*. *miltiorrhiza* extract may be added as it is, may be used in combination with other cosmetic ingredients, or may be appropriately used in accordance with conventional methods. The content of the active ingredient can be suitably determined according to the purpose of use thereof. In general, in the preparation of cosmetics using the S. *miltiorrhiza* extract, the active ingredient may be added in an amount of 0.0001 to 10% by weight, preferably 0.1 to 5% by weight, based on the total raw material weight. Cosmetics may be applied to skin water, lotion, creams, packs and make-up products, but are not limited thereto.

Although preparation examples have been disclosed, they are provided only for illustrative purposes rather than to limit the scope of the present invention. Those skilled in the art will appreciate that various modifications, additions and substitutions are possible, without departing from the scope and spirit of the invention as disclosed in the accompanying claims.

## Claims

1. A composition for preventing, ameliorating or treating visceral fat obesity containing a *Salvia miltiorrhiza* radix extract.

2. The composition according to claim 1, wherein the *Salvia miltiorrhiza* radix extract is extracted with any one solvent selected from the group consisting of water, alcohols having 1 to 6 carbon atoms and a mixed solvent thereof.

3. The composition according to claim 1 or 2, wherein the *Salvia miltiorrhiza* radix extract inhibits production of triglycerides (TGs) in liver tissue and blood, and reduces expression of alanine aminotransferase (ALT), glutamic pyruvate transaminase (GPT), lactate dehydrogenase (LDH), blood urea nitrogen (BUN), free fatty acid (FFA) or glycosylated Hb (Hb_{A1C}) when administered to high fat diet-induced obesity (DIO) mice.

4. The composition according to claim 1 or 2, wherein the *Salvia miltiorrhiza* radix extract causes visceral fat reduction and fat loss in liver tissue as observed through magnetic resonance imaging (MRI) and magnetic resonance spectroscopy (MRS) of fat and oil red O staining of liver tissue.

5. The composition according to claim 1, wherein the composition comprises a mixture of the *Salvia miltiorrhiza* radix extract and at least one mineral selected from chromium, manganese, magnesium, zinc, copper or calcium.

6. The composition according to claim 5, wherein the mineral comprises mineral diaminate or a mineral amino acid salt as a mineral source.

7. The composition according to claim 1, wherein the *Salvia miltiorrhiza* radix extract prevents, alleviates or treats a disease mediated by visceral fat selected from the group consisting of hypertriglyceridemia, fatty liver, hypertension, diabetes, hyperlipidemia, cardiocerebrovascular disease, abnormal glucose metabolism, abnormal blood coagulation or obesity.

8. A heath functional food composition for preventing or ameliorating visceral fat obesity containing a *Salvia miltiorrhiza* radix extract as an active ingredient.

9. The heath functional food composition according to claim 8, wherein the heath functional food composition contains the *Salvia miltiorrhiza* radix extract in an amount of 0.1 to 50% by weight.

10. A cosmetic composition comprising a *Salvia miltiorrhiza* radix extract as an active ingredient.
